Europäisches Patentamt

European Patent Office  (11) Veröffentlichungsnummer: **0 061 508**

Office européen des brevets  **B1**

(12) **EUROPÄISCHE PATENTSCHRIFT**

(45) Veröffentlichungstag der Patentschrift: **26.06.85**

(21) Anmeldenummer: **81102334.0**

(22) Anmeldetag: **27.03.81**

(51) Int. Cl.⁴: **A 61 K 35/64,** B 01 D 11/00, A 23 K 1/00, A 23 L 1/48, C 07 G 17/00

(54) **Verfahren zur Gewinnung von Bienenkittharz enthaltenden Produkten und Bienenkittharz enthaltende Produkte.**

(43) Veröffentlichungstag der Anmeldung: **06.10.82 Patentblatt 82/40**

(45) Bekanntmachung des Hinweises auf die Patenterteilung: **26.06.85 Patentblatt 85/26**

(84) Benannte Vertragsstaaten: **AT CH DE FR IT LI**

(56) Entgegenhaltungen: DE-B-1 037 651 LU-A- 69 108

**CHEMICAL ABSTRACTS, Band 71, Nr. 21, 24. November 1969, Seite 106, Nr. 99267t, Columbus, Ohio, U.S.A., S. SCHELLER et al.: "Antibacterial properties of propolis" CHEMICAL ABSTRACTS, Band 64, Nr. 5, 28. Februar 1966, Spalte 6403a, Columbus, Ohio, U.S.A., M.V. YALOVITSYN: "The effect of bee poison, honey, flower pollen, and bee glue on antibiotics and sulfanilamides activity"**

(73) Patentinhaber: **Schanze, Rudolf Friedenstrasse 43 D-8034 Unterpfaffenhofen (DE)**

(72) Erfinder: **Schanze, Rudolf Friedenstrasse 43 D-8034 Unterpfaffenhofen (DE)**

(74) Vertreter: **Kraus, Walter, Dr. et al Patentanwälte Kraus Weisert & Partner Thomas-Wimmer-Ring 15 D-8000 München 22 (DE)**

Courier Press, Leamington Spa, England.

EP 0 061 508 B1

# 0 061 508

**Beschreibung**

Die Erfindung betrifft ein Verfahren zur Herstellung von Bienenkittharz enthaltenden Produkten durch Extraktion von Bienenkittharz, das auch als Propolis bezeichnet wird, und die Produkte die Bienenkittharz enthalten.

Bienen sammeln an den Knospen und Wunden von Pflanzen, soweit an diesen Harz zutagetritt, dieses Material ein und kitten damit Ritzen im Stock und an der Beute (das sind die Waben und Wabenträger) aus. Außerdem überziehen sit mit dem von ihnen gesammelten und verarbeiteten Kittharz sowohl vorübergehend ungenutzte Waben und Rahmenteile, um diese zu desinfizieren und zu konservieren. Ferner werden Fremdkörper im Stock—z.B. Tier- und Insektenleichen, die in Verwesung übergehen können, mit Kittharz oder Propolis überzogen. Auf diese Weise schützen die Bienen sich und ihren unmittelbaren Lebensraum gegen bakterielle oder virusbedingte Infektionen, gegen Zersetzung und gegen Gefährdung.

Dieses von den Bienen erzeugte und verarbeitete Kittharz oder Propolis hat durchschnittlich die folgende Zusammensetzung:

| | | |
|---|---|---|
| etwa bis zu | 55% | Harze und Balsam |
| | 35% | Bienenwachs |
| | 10% | flüchtige Öle |
| | 5% | Flavonsubstanzen |
| | 5% | Pollen von Pflanzen. |

Bei der Veraschung dieses Kittharzes findet sich eine Reihe mineralischer Elemente, wie zum Beispiel: Al, Ca, Co, Cu, Fe, Mg, Mn, Na, Ni, P, Sr, Va, Zn und andere in unterschiedlichen und geringen Mengen.

Unter den organischen Stoffen des Kittharzes wird eine Reihe hochaktiver Stoffe gefunden, die desinfizierende, bakteriostatische, bakterizide, fungizide und antivirale Wirkungen aufweisen. Es finden sich ferner immunstimulatorische Substanzen und Stoffe, die im Stoffwechsel von Lebewesen besonders günstige Wirkungen aufweisen. Solche Substanzen sind bereits definiert und quantifiziert, analytisch bestimmt und benannt worden. Von ihnen seien einige aufgezählt: Flavone, Flavonole, Flavonone, wie z.B. Chrysin, Galangin, Ramnocytrin; Terpene, wie z.B. Geraniol und Nerol; organische Säuren, wie Sorbinsäure, Benzoesäure, Myristinsäure, Kaffeesäure, Ferulasäure und Zimtsäure.

Es ist bekannt, daß die Anwendung von Kittharz in natürlichen Mitteln und in der Naturheilkunde als Vorbeugungsmittel und als Mittel zur Gesunderhaltung bei Mensch und Tier äußerst günstige Ergebnisse zeigt.

Präparate, die Bienenkittharz enthalten, wirken grundsätzlich gegen eine Reihe bakterieller und viraler Affekte. Besonders gut ist die Wirkung bei gesundheitlichen Störungen im Hals-Nasen-Ohren-Bereich, im Mund und Rachenraum. Ferner wird über günstige Wirkungen bei Störungen im Magen- und Darmtrakt berichtet, insbesondere beobachtet man synergistische Wirkung in Verbindung mit bestimmten Arzneimitteln. Bei Virusgrippen und Lungenstörungen hat man ebenfalls starke Besserungen beobachtet. Im Dentalbereich stellt sich eine anästhesierende Wirkung ein, die nach einigen Berichten derjenigen üblicher Anästhetika beispielsweise Novokain überlegen sein soll.

Diese Wirkungen sind auf die zuvor erwähnten Inhaltestoffe zurückzuführen, die im Kittharz enthalten sind und auf andere Stoffe, welche von den Bienen stammen, wie Wachs, Pollen und Honig, und quasi als Verunreinigung in dem Kittharz enthalten sind und mit diesem zusammen gewonnen werden.

Kittharz ist somit eine gesundheitsfördernde und -stützende Substanz, wobei im Vordergrund steht, daß schädliche Nebenwirkungen nicht beobachtet wurden. Die toxikologischen Eigenschaften von Kittharz sind erforscht und geprüft. So beträgt für die weiße Labormaus die $LD_{50}$ 0,7 mg/g bei subcutaner Anwendung und für die Katze 0,1 mg/g. Sie liegt damit so hoch, daß sie in der Praxis nicht realisiert werden kann. Kittharz ist somit als Nahrungsbestandteil unbedenklich.

Wegen dieser außerordentlich vorteilhaften Eigenschaften: Förderung von Widerstandskraft und Gesundheit ohne Nebenwirkungen und Risiko, findet Kittharz zunehmendes Interesse und sich schnell verbreitende Anwendung.

Allerdings ist die Gewinnung von Kittharz schwierig. Einmal sind die beschaffbaren Mengen gering, zum anderen tritt bei der Gewinnung des Kittharzes aus den Bienenstöcken eine mehr oder weniger starke Verunreinigung mit anhaftendem Material auf. Die Verunreinigungen betreffen sowohl anhaftende andere Bienenprodukte, wie Wachs, Pollen und Honig, die nicht nur unbedenklich sind, sondern die sogar wegen ihrer günstigen Eigenschaften erwünschte sind, sie betreffen aber auch Holzteilchen und Schmutz, die entfernt werden müssen.

Bei den bekannten Verfahren wird daher die spezielle Kittsubstanz gegebenenfalls mit den geringen Anteilen an Wachs, Pollen und Honig, d.h. die Bienenprodukte von bienenfremden Bestandteilen, wie Holz

2

und anderen festen, unlöslichen Partikeln getrennt, indem man unter Verwendung von Lösungsmitteln eine Extraktion durchführt und die erhaltene Lösung konzentriert.

Als Extraktionsmittel verwendet man üblicherweise Alkohole, bevorzugt Äthylalkohol, oder Wasser-Alkohol-Gemische. Die Extraktion von Bienenkittharz erfolgt im allgemeinen bei erhöhter Temperatur und man erhält alkoholische oder wässrig-alkoholische Extrakte mit 5 bis 20 Anteilen von Kittharz.

Da Alkohol als solcher ein starkes Zellgift ist und auch in geringer Dosierung und Verdünnung das Zellgewebe von Lebewesen schädigt, zumindest reizt, ist seine Verwendung nicht unbedenklich, insbesondere dann, wenn man die erhaltenen Extrakte ohne Entfernung des Extraktionsmittels verwenden will.

In der DE—AS 10 37 651 wird ein Verfahren zur Herstellung eines therapeutisch wirksamen Extraktes aus Propolis beschrieben. Bei diesem bekannten Verfahren wird verdünnte Alkalilauge als Extraktionsmittel verwendet. Anstelle von rein wässrigen alkalisch reagierenden Lösungsmitteln, können auch wässrige alkalische Extraktionsmittel in Mischung mit wasserlöslichen organischen Verbindungen verwendet werden. Bei diesem bekannten Verfahren erhält man eine bräunlich gefärbte, amorphe, hygroskopische Substanz, die einen intensiven Geruch aufweist. Die bei dem bekannten Verfahren erhaltene Substanz muß jedoch weiter gereinigt werden, bevor sie verwendet werden kann. Dies ist für eine Herstellung des Extrakts in großem Maßstab nachteilig.

Der vorliegenden Erfindung liegt somit die Aufgabe zugrunde, ein Extraktionsverfahren zur Verfügung zu stellen, mit dem das Bienenkittharz gewonnen werden kann und bei dem es nicht erforderlich ist, Alkohol zu verwenden. Das erfindungsgemäße Verfahren soll auf einfache Weise und wirtschaftlich durchzuführen sein. Die Ausbeute an Bienenkittharz soll auch möglichst hoch sein.

Überraschenderweise wurde gefunden, daß Cholin, bestimmte Cholinderivate, wie z.B. sein Oxidationsprodukt, das Betain oder Gemische dieser Verbindungen, z.B. ein Gemisch aus Cholin-Base und Cholin-Chlorid für die Extraktion von Kittharz (Bienenprodukt) gut geeignet ist. Durch die Verwendung dieser Extraktionsmittel werden die unerwünschten Eigenschaften von Alkohol bei der Extraktion von Bienenkittharz vermieden.

Die Erfindung betrifft ein Verfahren zur Gewinnung von Bienenkittharz enthaltenden Produkten durch Extraktion, das dadurch gekennzeichnet ist, daß man zur Extraktion Cholin und/oder Cholinderivate oder wässrige Lösungen hiervon einsetzt.

Cholin, Trimethyloxyäthyl-ammonium-hydroxid, ist eine sehr starke organische Base, die bei der Spaltung von Lecithin entsteht. Sie ist in Wasser und Alkohol leicht löslich und in der Natur weit verbreitet. Beispielsweise findet man Cholin in Gehirnextrakt, Eigelb, Pilzen, insbesondere in den Steinpilzen und Champignons, aber es tritt auch im Hopfen auf.

Cholin wirkt blutdrucksenkend. Es vermindert die Fettablagerung im Körper. Auch die Darmschleimhaut enthält viel Cholin, weshalb man es auch als Hormon der Darmbewegung bezeichnet hat. Es regt die Tätigkeit der glatten, vegetativen Muskulatur an. Cholin findet in Arzneimitteln Anwendung gegen Arterienverkalkung und zur Beseitigung von Leberparenchymschäden.

Auch in der Tierernährung wird Cholin, insbesondere bei Geflügel und Schweinen, sowie bei Kälbern, in größeren Mengen als Wirkstoff eingesetzt, um gegen Leberbelastungen und im Kohlehydrat- und Fettstoffwechsel fördernd und stützend zu wirken. Cholin ist somit ein hilfreiches und lebensnotwendiges und gesundheitsförderndes Mittel und ergänzt die Eigenschaften des Kittharzes sehr gut.

Als andere Cholinderivate kann man beispielsweise physiologisch verträgliche Cholinsalze, z.B. das Cholin-Chlorid oder Betain, das durch Oxidation aus dem Cholin gewonnene Hydrat des Trimethylglykols, welches teilweise dem Cholin überlegene Stoffwechseleigenschaften aufweist, verwenden. Betain ergänzt das Wirkungsspektrum der bereits genannten Cholinderivate sinnvoll. Insbesondere weil es beim Ersatz von Cholin die nachfolgende Reaktion nach rechts verschiebt.

Homocystin+Cholin⇌Methionin+Dimethylaminäthanol.

Betain und Vitamin-B-12 können sich bezüglich der Wachstumförderung gegenseitig ergänzen. Bei Argininmangel wird Betain in der Kreatinsynthese wirksam, wodurch ebenfalls das Wachstum verbessert wird. Eine Reihe von Stoffwechselstörungen beruht oft weniger auf Cholinmangel, als auf ungenügender Enzymoxidation von Cholin zu Betain, damit ist Betain ein besserer Methyldonator bei der Transaminierung. Die positiven Wirkungen von Betain sind durch entsprechende Literaturberichte aus Tierversuchen erhärtet. Betain ist somit innerhalb des Systems ein weiterer wertvoller Faktor der Unterstützung in der Wirksamkeit der Extrakte und bei ihrer technischen Darstellung.

Das Cholin hat einen extrem hohen pH-Wert, der im Bereich von pH 14 liegt. Erfindungsgemäß kann man bei diesem hohen pH-Wert arbeiten, bevorzugt arbeitet man jedoch bei einem pH-Wert im Bereich von 10 bis 13, besonders bevorzugt im Bereich von 11,5±0,5.

Cholinhydrochlorid besitzt einen pH-Wert im Bereich von 6 bis 7. Um erfindungsgemäß einen Bereich von 10 bis 13 zu erreichen, verwendet man ein Gemisch aus Cholin und Cholinhydrochlorid. Die Bestandteile werden so verwendet, daß man ein Gemisch mit einem pH-Wert im Bereich von 10 bis 13, bevorzugt von 11 bis 12 und besonders bevorzugt von 11,5±0,3 erhält. Bei der praktischen Durchführung der Extraktion legt man Cholin-Chlorid, welches im Handel als 70%-ige wässrige Lösung erhältlich ist und einen pH-Wert von ca. 6,8 besitzt, vor und titriert mit Cholin-Base, die im Handel als 50%-ige wässrige Lösung erhalten ist, auf einen pH-Wert im Bereich von 10 bis 13 und besonders bevorzugt auf einen pH-Wert von 11,5±0,3.

In Abhängigkeit von dem gewünschten pH-Wert erhält man so eine Mischung aus Cholin-Base und Cholin-Chlorid. Im bevorzugten pH-Bereich liegt das Verhältnis zwischen Cholin-Chlorid zu Cholin-Base zwischen 6:1 bis 10:1.

Betain besitzt als wässrige Lösung einen pH-Wert von 9 bis 10. Betain kann alleine zur Extraktion verwendet werden, d.h. man kann die Extraktion bei dem pH-Wert des Betains durchführen. Vorzugsweise verwendet man jedoch das Betain zusammen mit Cholin oder mit dem oben beschriebenen Gemisch aus Cholin-Base und Cholin-Chlorid. Bevorzugt verwendet man solche Gemische bei den oben angegebenen pH-Bereichen. Wird in das Lösungsmittelsystem aus Cholin und Cholin-Chlorid noch Betain eingeführt, so verwendet man bevorzugt Verhältnisse zwischen Cholin-Chlorid:Betain:Cholin-Base, die im Bereich von 4:2:1 bis zu 4:6:1 liegen.

Bei der Extraktion des Kittharzes soll nicht nur dieses isoliert werden, sondern es sollen auch andere nützliche Substanzen, die in Kittharz als Nebenprodukte vorhanden sind, wie die oben erwähnten Bienenprodukte, isoliert werden. Man gibt deshal vorzugsweise bei der Extraktion bekannte Emulgatoren, wie beispielsweise Polyglycerin-Stearat hinzu.

Die durch Emulgatoren erhöhte Wirksamkeit fettorientierte Extraktstoffe gegenüber der anteiligen Wasserphase in Verteilung und Emulsion zu halten, wirkt sich positiv sowohl auf die Homogenität des Endproduktes, als auch auf die in der Extraktion überführte Menge an Extraktsstoffen—kurz auf die Ausbeute—aus und erhöht dadurch die Wirtschaftlichkeit des Verfahrens beträchtlich.

Bei der Extraktion kann man die Emulgatoren verweden, die man normalerweise auch bei der Extraktion von Bienenkittharz unter Verwendung von Alkohol als Extraktionsmittel eingesetzt hat. Überraschenderweise hat sich gezeigt, daß diese Emulgatoren auch im stark basischen Bereich, bei dem erfindungsgemäß die Extraktion durchgeführt wird, wirksam sind.

Die erfindungsgemäße Extraktion wird im basischen und stark basischen Bereich durchgeführt und man erhält dabei eine sehr gute Lösung und Dispersion der Kittharzbestandteile. Es ist möglich, 5 bis 25% des Kittharzes in Lösung und Dispersion zu überführen. Die Ausbeute ist somit wesentlich höher als bei dem bekannten Verfahren.

Abhängig von der Qualität des Kittharzes und dem Verhältnis zwischen auszubeutenden Anteilen und ihren Verunreinigungen schwankt das Extraktionsergebnis mehr oder weniger stark. Im Schnitt liegt der Extraktanteil bei Alkohol bei über 0—20%, mit Cholinderivaten bei 5—25%, was eine relative Verbesserung von ca. +15—30% ausmacht, verglichen mit Alkoholextraktion.

Nach Beendigung der Extraktion wird das unlösliche Material durch Filtration abgetrennt. Man kann erfindungsgemäß auch mehrere Extraktionsstufen miteinander kombinieren und das bei der ersten Filtration erhaltene Filtrat als weiteres Extraktionsmittel für noch nicht behandeltes Bienenkittharz verwenden und das bei der ersten Extraktion erhaltene unlösliche Material mit neuem Extraktionsmittel extrahieren. Dies läßt sich beliebig wiederholen, so daß man die Extraktion auch in mehreren Stufen durchführen kann, wobei jedesmal das Extraktionsmittel zur Extraktion des unlöslichen Materials, das bei der darauffolgenden Stufe erhalten wird, verwendet wird und das erhaltene Filtrat zur Extraktion des festen Materials, das man bei der vorherigen Stufe erhält, verwendet wird.

Die erfindungsgemäße Extraktion wird im allgemeinen bei Temperaturen im Bereich von 50 bis 70°C, bevorzugt zwischen 55 bis 65°C, besonders bevorzugt bei 60°C durchgeführt. Sie kann jedoch auch bei Zimmertemperatur durchgeführt werden. Die Extraktionszeiten hängen von der verwendeten Temperatur ab und betragen im allgemeinen 30 Minuten bis 6 Stunden.

Die Filtration erfolgt bei etwas niedrigerer Temperatur, im allgemeinen in einem Bereich von 30 bis 50°C, bevorzugt in einem Bereich von 35 bis 45°C, besonders bevorzugt bei 40°C. Dies gilt auch für die wiederholte Extraktion und Filtration.

Nach Abschluß des Extraktions- und Filtrationsverfahrens erhält man einen Extrakt, der im allgemeinen einen hohen pH-Wert, der im Bereich zwischen 10 und 13 liegt, aufweist. Aus Gründen der besseren Applikation und des Geschmackes ist es wünschenswert, diesen pH auf einen Wert zu erniedrigen, der physiologisch, geschmacklich und applikationsmäßig günstiger ist.

Bei der Erniedrigung des pH-Wertes muß jedoch eine Ausfällung oder die Abscheidung eines Bodensatzes (Sedimentierung) vermieden werden und außerdem muß die Wirksamkeit der Emulsion erhalten bleiben. Zur Erniedrigung des pH-Wertes muß daher eine physiologisch annehmbare Substanz verwendet werden, wie z.B. anorganische oder organische Säuren. Beispiele für anorganische Säuren sind die Phosphorsäure und die Schwefelsäure und Beispiele für organische Säuren sind Ascorbin-, Zitronen-, Essig-, Propion-, Zimt-, Kaffee-, Ferula- und Myristinsäure. Es können jedoch auch andere Säuren verwendet werden.

Die sauren Substanzen werden dem Extrakt zugegeben, bis man einen pH-Wert erreicht, der im Bereich zwischen 6,5 bis 9,5, vorzugsweise im Bereich von 7 bis 8,5, besonders bevorzugt im Bereich von 7,6±0,3 liegt. In diesen Bereichen wird einerseits das Lösungs- und Dispersionsvermögen der gelösten bzw. dispersierten Substanzen beibehalten, andererseits erhält man einen Extrakt, der eine besonders gute physiologische Verträglichkeit und Anwendungsmöglichkeit besitzt.

Wie zuvor erwähnt, enthält Kittharz verschiedene Bestandteile und bestizt gesundheitsfördernde, widerstandskräftigende Eigenschaften ohne schädliche Nebenwirkungen. Es ist jedoch ein Naturprodukt

# 0 061 508

und daher wird seine endgültige Zusammensetzung von der Vegetation, dem Klima, dem Standort und der Jahreszeit und außerdem von den Bienenarten, von denen es gesammelt wird, bestimmt.

Es ist jedoch für den Hersteller und den Verbraucher von Interesse, daß der aus dem Bienenkittharz erhaltene Extrakt im allgemeinen eine gleiche Zusammensetzung aufweist und daß keine zu großen Qualitätsunterschiede in dem Extrakt auftreten.

Die wesentlichsten wirksamen Inhaltsstoffe von Kittharz sind:

| | |
|---|---|
| Ferula-Säure | Zimt-Säure und Derivate |
| Quercetin | Kaffee-Säure und Derivate |
| Geraniol | Myristin-Säure und Derivate |
| Chrysin | Galangin |

Erfindungsgemäß wird ein Gemisch aus den Inhaltsstoffen hergestellt und dieses Gemisch, das als Standardisierungsmischung bezeichnet wird, ja nach Bedarf zu dem erhaltenen Extrakt gegeben.

Das Gemisch enthält folgende Bestandteile:

| | |
|---|---|
| 20—60 Gew.%, vorzugsw. 30—50 Gew.% | Myristinsäure |
| 10—50 Gew.%, vorzugsw. 20—40 Gew.% | Zimtsäure |
| 3—15 Gew.% | Quercetin |
| 3—15 Gew.% | Geraniol |
| 0—5 Gew.% | Ferula-Säure |
| 0—7,5 Gew.% | Chrysin |
| 0—10 Gew.% | Kaffeesäure |

bezogen auf die Mischung.

Erfindungsgemäß wird dieses Gemisch zu dem Extrakt zugegeben, sofern er diese Inhaltsstoffe des Kittharzes nicht in ausreichender Menge enthält. Ein Beispiel für ein solches Gemenge zur Standardisierung ist die im folgenden beschriebene Mischung:

Dieses oben erwähnte Gemisch kann durch Zugabe weiterer Zusatzstoffe wie Vitamine, Spurenelemente, Mineralstoffe, Stabilisatoren, Aromastoffe, Aminosäuren oder pharmakologisch wirkende Stoffe oder ihre Gemische angereichert werden.

Der pH-Wert des erhaltenen Extraktes kann vor oder nach Standardisierung, wie oben ausgeführt, eingestellt werden.

Standardisierungszusatzmischung für Kittharz-extrakte aus Cholinderivaten ist beispielsweise eine Mischung, die sich wie folgt zusammensetzt:

| | |
|---|---|
| 368,5 g | Myristinsäure |
| 250 | Zimtsäure |
| 62,5 | Quercetin |
| 62,5 | Geraniol |
| 2,5 | Ferula-Säure |
| 2,5 | Chrysin |
| 1,5 | Kaffeesäure |
| 750 g | Mischung |

Der gesamte Arbeitsgang kann somit schematisch wie folgt dargestellt werden:

1) Herstellung des Extraktionsmittels aus Cholin- und/oder Cholinderivaten, Emulgatoren und/oder Wasser mit eingestelltem pH-Wert;

2) Zugabe des Kittharzes;

5

**0 061 508**

3) ein- bis mehrmalige Extraktion in vorgebenen bestimmten Temperaturbereichen;

4) anschließende ein- bis mehrmalige Filtration in bestimmten Temperaturbereichen;

5) Einstellung des gewünschten End-pH-Wertes durch sauer reagierende Verbindungen;

6) gegebenenfalls Standardisierung der Inhaltsstoffe durch Zugabe von einem Gemisch aus Wirkstoffen nach der Filtration.

Zu dem erhaltenen Extrakt, der gegebenenfalls auch eine Standardisierungszusatzmischung enthält, kann man andere, weitere Wirkstoffe oder Zusatztstoffe, wie beispielsweise Vitamine, Aromastoffe, Spurenelemente, Mineralstoffe, Geschmacksverbesserungsmittel oder Pharmazeutika geben.

Verwendet man Cholin und/oder Cholinderivate wie Betain als Extraktionsmittel bei der Aufarbeitung des Kittharzes, so läßt sich das Kittharz auf wirtschaftlichere Weise herstellen, da das Cholin günstigen zugänglich ist wie der Alkohol, und weiterhin wird bei der Extraktion die schädliche Wirkung des Alkohols auf die Zellen vermieden.

Es ist ein weiterer Vorteil des erfindungsgemäßen Verfahrens, daß die Einstellung des gewünschten pH-Wertes zur Extraktion aus Cholinderivaten, die selbst unterschiedliche pH-Werte aufweisen, erreicht werden kann. Man kann so als Extraktionsmittel ausschließlich körperfreundliche und gesundheitswirksame Substanzen verwenden.

Die erhaltenen Extrakte können als solche in flüssiger Form vermarktet und dem Endverbraucher zugeführt werden. Man kann sie jedoch auch als Halbfabrikate verwenden und einer Weiterverarbeitung zuführen. Beispielsweise kann man sie als Träger für Medikamente einsetzen oder man kann sie mit Lebensmitteln oder Naturpräparaten (Honig, Fruchtsaft, Sirup) vermengen. Die erfindungsgemäßen Produkte lassen sich weiter durch Zusatz von pulverförmigen Siliciumdioxid in pastose bis trockene fließfähige Formen—in an sich bekannter Weise- überführen.

Aus dem erhaltenen Extrakt kann man auch einen Teil des Lösungsmittels entfernen, um konzentrierte Lösungen zu erhalten. Sowohl die flüssigen Extrakte, wie auch die mischfähig gemachten Trockenprodukte können direkt verwendet werden oder sie können in Nahrungsmittel, Futtermittel für Mensch und Tier gegebenenfalls nach Aromatisierung und Geschmackskorrektur eingearbeitet werden. Sie können auch mit weiteren Bienenprodukten vermischt werden.

Soll das Kittharzextrakt direkt verwendet werden, so können Geruch und Geschmack der Cholinderivate nachteilig sein. Daher ist es förderlich diese Sinneswahrnehmungen zu verringern und zu verändern.

Dies wird erreicht, wenn man den gewonnenen Extrakt mit geschmacksaktiven Genuß- und/oder Lebensmitteln, wie beispielsweise verschiedenen Zuckern (z.B. Fructose, Saccharose), Kakao- und Malz- oder Kaffeepulver oder Kakao-, Kaffee- und Malzextrakten, vermischt oder aber Sellerie-, Kapern oder anderen Frucht- oder Gemüsekonzentraten vermischt und diese mit beispielsweise Zimt, Wermut, Muskat würzt. Damit können die Geschmacksrichtungen geändert und unangenehmer Beigeschmack des Kittharzextraktes überdeckt werden.

Besonders vorteilhaft ist es, zuvor mit geringen Anteilen an Aktivkohle den Geruch zu adsorbieren. Dies geschieht zweckmäßigerweise zuvor, indem zuerst eine geringe Menge Aktivkohle zu dem Extrakt gibt und erst dann die weiteren korrigierenden Zusätze verwendet, sodaß damit eine sämige bis pastose Konsistenz erreicht wird, welche die Einnahme erleichtert und verbessert.

Das bei der erfindungsgemäßen Extraktion erhaltene Bienenkittharz enthaltende Produkt enthält 5 bis 40, vorzugsweise 10 bis 30 Gew.% Bienenkittharz (einschließlich der aus den Bienenprodukten stammenden Begleitsstoffe), 60 bis 95, vorzugsweise 30 bis 50 Gew.% Cholin und/oder Cholinderivate bezogen auf das Gemisch aus Bienenkittharz, Cholin und/oder Cholinderivate und bis zu 30 Gew.% Wasser bezogen auf das Gesamtgemisch.

Dieses Produkt kann bis zu 15 Gew.% zusätzlich Vitamine, Spurenelemente, Mineralstoffe, Stabilisatoren, Aromastoffe, Aminosäuren und/oder pharmakologisch wirkende Stoffe oder ihre Gemische enthalten. Es kann weiterhin zusätzlich bis zu 5 Gew.% der zuvor beschriebenen Standardisierungsmischung enthalten.

Gibt man zu dem Bienenkittharzextrakt die oben erwähnten Zusatzstoffe, so verwendet man die in der folgenden Tabelle aufgeführten Gew.-%-Gehalte.

# 0 061 508

TABELLE I
Verschiedene Rezeptoren für handelsübliche Produkte

| Bestandteil | Gew.%<br>allgem. Bereich | Gew.%<br>bevorz. Bereich |
|---|---|---|
| 1 Bienenkittharzextrakt | 30—70 | 40—60 |
| 2 Aktivkohle | 0—20 | 0—15 |
| 3 Kakao-/Kaffee-/Malzpulver und/oder -extrakte | 20—50 | 25—45 |
| 4 Zucker | 0—25 | 0—20 |
| 5 Gewürze | 0—5 | 0—4 |

Die folgenden Beispiele erläutern die Erfindung.

Beispiel 1
Extraktion von Kittharz

Wie in der folgenden Tabelle II schematisch zusammengestellt, wird ein Extraktionsmittel aus Cholin-Base, Cholinchlorid und Emulgator hergestellt. Zu diesem Extraktionsmittel wird das Kittharzgemenge zugegeben. Die Zugabe des Kittharzes erfolgt bei der Temperatur von 60°C. Während dreißig Minuten wird dann bei dieser Temperatur gerührt, anschließend läßt man das Gemisch auf 40°C abkühlen und filtriert. Dann wird erneut extrahiert und nochmals werden dreißig Minuten gerührt. Nach der Filtration erhält man das Endprodukt.

TABELLE II

| Bearbeitung | Volumen Liter | Masse kg | Trockenm. kg | Temp. °C | pH | Legende |
|---|---|---|---|---|---|---|
| Extraktionsmittel | | | | | | |
| A | 84,5 | 84,5 | 59,15 | 60 | 6,8 | Cholin-Chlorid 70%ig, wässr. Lösg.; |
| B | 9 | 9 | 4,50 | 60 | 14 | Cholin-Base 50%ig, wässr. Lösg. |
| C | 2,5 | 2,5 | 2,5 | 60 | | Emulgator Polyglycerin-Stearat |
| mischen | | | | 60 | 11,5 | Extraktionsgemenge |
| Zugabe | 15 | 15 | 15 | 60 | 11,5 | Rohmaterial Kittharz-gemenge, unrein |
| während 30 Min. stetig rühren (digerieren) abkühlen | | | | 40 | 11,5 | |
| Filtrieren und nochmals extrahieren (erwärmt) | | | | | | |
| Extraktionsmittel A+B+C | 50 | 50 | 34,45 | 60 | 11,5 | erneute Extraktion des Filterrückstandes |
| während 30 Min. stetig rühren (digerieren) abkühlen | | | | 40 | 11,5 | |
| Filtrieren | | | | | | |
| Zugabe | 4 | 4,6 | 0,78 | 40 | 7,8 | Phosphorsäure 17%ig |

Das gewonnene Endprodukt enthält 70% aktive Substanzen davon 12 bis 13% Kittharzextrakte/dispergate und 30% Restwasser.

7

Beispiel 2
Extraktion und Standardisierung von Kittharz

Man arbeitet, wie im Beispiel 1 beschrieben, wobei man die in der folgenden Tabelle III aufgeführten Bedingungen verwendet:

### TABELLE III

| Bearbeitung | Volumen Liter | Masse kg | Trockenm. kg | Temp. °C | pH | Legende |
|---|---|---|---|---|---|---|
| Extraktionsmittel wie Beispiel 1 gemischt erwärmt | 97 | 97 | 67,15 | 60 | 11,5 | aus Cholin-Chlorid (70%) +Cholin-Base (50%)+ Emulgator, pH-korrigiert |
| Zugabe | 25 | 25 | 25 | 60 | 11,5 | Rohmaterial, Kittharz-gemenge, unrein |
| während 45 Min. stetig rühren (digerieren) Temperatur wie vorher abkühlen | | | | 40 | 11,5 | |
| Filtrieren | | | | | | |
| Zugabe | 0,75 | 0,75 | 0,75 | 40 | 11,5 | Standardisierungs-zusatz gemäß dem angegebenen Rezept |
| mischen, homogene Verteilung abwarten | | | | | | |
| Zugabe | 4 | 4,6 | 0,78 | 40 | 7,8 | Phosphorsäure 17%ig |

Das gewonnene Endprodukt enthält 73,5% aktive Substanzen davon 26—27% Kittharz, 26,4% Restwasser und ist in 7 Wirkstoffen quantitativ standardisiert.

Beispiele 3 bis 6

Zur Herstellung von gut genießbaren Gemischen aus dem Bienenkittharzextrakt, wie es gemäß den vorherigen Beispielen erhalten wurde, wurden zu dem Bienenkittharzextrakt verschiedene Geschmacks- und Aromastoffe, wie in der folgenden Tabelle IV angegeben, zugegeben.

Man erhält bei allen Beispielen angenehm schmeckende Produkte, die für die menschliche und tierische Ernährung geeignet sind.

### TABELLE IV
Gewichtsanteile der Endmischungen in %

| Bestandteile | 3 | 4 | 5 | 6 | 7 | 8 |
|---|---|---|---|---|---|---|
| Kittharzextrakt mit Cholinderivaten | 40 | 45 | 40 | 55 | 60 | 50 |
| Aktivkohle | — | 8 | 15 | 10 | 5 | 3 |
| Kakao/Kaffe/Malzpulver oder -extrakt | — | — | 25 | 27 | 30 | 35 |
| Gemüse und/oder Obst-konzentrate (Sellerie, Kapern, Zitrus) | 50 | 45 | — | — | — | — |
| Gewürze (Zimt, Muskat, Wermut) | 2 | 2 | 1 | 2 | — | 1 |
| Zucker (Fructose, Saccharose, Malzextrakte) | 8 | — | 19 | 6 | 5 | 11 |

# 0 061 508

**Patentansprüche**

1. Verfahren zur Gewinnung von Bienenkittharz enthaltenden Produkten durch Extraktion von natürlichen Bienen kittharz, dadurch gekennzeichnet, daß man zur Extraktion des Bienenkittharzes Cholin und/oder Cholinderivate oder wässrige Lösungen hiervon einsetzt.

2. Verfahren nach Anspruch 1, dadurch gekennzeichnet, daß man Cholin und ein CHolinderivat zunächst so miteinander mischt, daß sich ein pH-Wert von 10 bis 13, bevorzugt jedoch 11 bis 12 einstellt, mit diesem Gemisch extrahiert und anschließend mit physiologisch verträglichen anorganischen und/oder organischen Säuren den pH-Wert des Extrakts auf einen Wert von 6,5 bis 9,5, bevorzugt 7 bis 8,5 einstellt.

3. Verfahren nach einem der Ansprüche 1 oder 2, dadurch gekennzeichnet, daß als Cholinderivate Cholin-Chlorid und/oder Betain verwendet werden.

4. Verfahren nach einem der Ansprüche 1 bis 3, dadurch gekennzeichnet, daß die Extraktion des Bienenkittharzes in einem Temperaturbereich zwischen 50 bis 70°C, bevorzugt 55 bis 65°C und die Filtration des Extraktes in einem Temperaturbereich von 30 bis 50°C, bevorzugt 35 bis 45°C, vorgenommen wird.

5. Verfahren nach einem der Ansprüche 1, 2, 3 oder 4, dadurch gekennzeichnet, daß der pH-Wert des erhaltenen Extraktes mit einer physiologisch annehmbaren, anorganischen oder organischen Säure auf einen Bereich von 6,5 bis 9,5 eingestellt wird.

6. Verfahren nach Anspruch 2 oder 5, dadurch gekennzeichnet, daß man zur pH-Einstellung Phosphorsäure, Schwefelsäure, Ascorbinsäure, Zitronensäure, Essigsäure, Propionsäure, Zimtsäure, Kaffeesäure, Ferulasäure, Myristinsäure oder Gemische hiervon verwendet.

7. Verfahren nach einem der Ansprüche 1, 2, 3, 4, 5 oder 6, dadurch gekennzeichnet, daß man bei der Extraktion einen Emulgator verwendet.

8. Bienenkittharz enthaltendes Produkt, dadurch gekennzeichnet, daß es 5 bis 40 Gew.-% Bienenkittharz, 60 bis 95 Gew.-% Cholin und/oder Cholinderivate, bezogen auf das Gemisch aus Bienenkittharz, Cholin und/oder Cholinderivaten, sowie gegebenenfalls bis zu 30 Gew.-% Wasser bezogen auf das Gesamtgemisch enthält.

9. Bienenkittharz enthaltendes Produkt, dadurch gekennzeichnet, daß es 30 bis 70 Gew.-% Bienenkittharzextrakt nach Anspruch 8, 0 bis 20 Gew.-% Aktivkohle, 20 bis 50 Gew.-% Kakao-, Kaffee-, Malzpulver und/oder -extrakt oder Gemüse- und/oder Obstkonzentrat, 0 bis 25 Gew.-% Zucker, 0 bis 5 Gew.-% Gewürze enthält.

10. Bienenkittharz enthaltendes Produkt nach Anspruch 8 und 9, dadurch gekennzeichnet, daß es bis zu 15 Gew.-% bezogen auf das Gesamtgemisch Vitamine, Spurenelemente, Mineralstoffe, Stabilisatoren, Aromastoffe, Aminosäuren und/oder pharmakologisch wirkende Stoffe oder ihre Gemische enthält.

11. Bienenkittharz enthaltendes Produkt nach Anspruch 8, 9 oder 10, dadurch gekennzeichnet, daß es zusätzlich bis zu 5 Gew.% einer Standardisierungszusatz mischung enthält, die folgende Zusammensetzung besitzt:

20 bis 60 Gew.-% Myristinsäure,
10 bis 50 Gew.-% Zimtsäure,
3 bis 15 Gew.-% Quercetin,
3 bis 15 Gew.-% Geraniol,
0 bis 5 Gew.-% Ferula-Säure,
0 bis 7,5 Gew.-% Chrysin,
9 bis 10 Gew.-% Kaffeesäure.

**Revendications**

1. Procédé d'obtention de produits contenant du propolis par extraction de propolis naturel, caractérisé en ce que l'on utilise pour l'extraction du propolis, la choline et/ou des dérivés de la choline ou leurs solutions aqueuses.

2. Procédé selon la revendication 1, caractérisé en ce que l'on mélange tout d'abord entre eux de la choline et un dérivé de choline de façon telle qu'il s'établisse un pH compris entre 10 et 13, mais de préférence cependant compris entre 11 et 12, en ce que l'on extrait à l'aide de ce mélange et qu'on ajuste ensuite le pH de l'extrait à une valeur comprise entre 6,5 et 9,5, et de préférence comprise entre 7 et 8,5, à l'aide d'acides minéraux et/ou organiques physiologiquement compatibles.

3. Procédé selon l'une des revendications 1 ou 2, caractérisé en ce que l'on utilise comme dérivés de choline, le chlorure de choline et/ou la bétaïne.

4. Procédé selon l'une quelconque des revendications 1 à 3, caractérisé en ce que l'on effectue l'extraction du propolis dans un intervalle de températures compris entre 50 et 70°C, et de préférence entre 55 et 65°C et en ce que l'on effectue la filtration de l'extrait dans un intervalle de températures compris entre 30 et 50°C, et de préférence entre 35 et 45°C.

5. Procédé selon l'une des revendications 1, 2, 3 ou 4, caractérisé en ce que l'on ajuste le pH de l'extrait obtenu dans la zone de pH comprise entre 6,5 et 9,5, à l'aide d'un acide organique ou minéral physiologiquement compatible.

6. Procédé selon l'une des revendications 2 ou 5, caractérisé en ce qu'on utilise pour ajuster le pH, l'acide phosphorique, l'acide sulfurique, l'acide ascorbique, l'acide citrique, l'acide acétique, l'acide

**0 061 508**

propionique, l'acide cinnamique, l'acide caféique, l'acide férulique, l'acide myristique ou des mélanges de ceux-ci.

7. Procédé selon l'une des revendications 1, 2, 3, 4, 5 ou 6, caractérisé en ce que l'on utilise un émulsifiant lors de l'extraction.

8. Produit contenant du propolis, caractérisé en ce qu'il contient:

5 à 40% en poids de propolis,

60 à 95% en poids de choline et/ou de dérivés de choline, rapportés au mélange de propolis, de choline et/ou de dérivés de choline, ainsi qu'éventuellement jusqu'à 30% en poids d'eau par rapport au mélange total.

9. Produit contenant du propolis, caractérisé en ce qu'il contient:

30 à 70% en poids d'extrait de propolis selon la revendication 8,

0 à 20% en poids de charbon actif,

20 à 50% en poids de poudre et/ou d'extrait de cacao, de café ou de malt ou de concentré de légumes et/ou de fruits,

0 à 25% en poids de sucre et,

0 à 5% en poids d'épices.

10. Produit contenant du propolis selon les revendications 8 et 9, caractérisé en ce qu'il contient jusqu'à 15% en poids, par rapport au mélange total, de vitamines, d'oligoéléments, de matières minérales, de stabilisants, d'aromates, d'amino-acides et/ou de substances présentant une activité pharmacologique ou de leurs mélanges.

11. Produit contenant du propolis selon la revendication 8, 9 ou 10, caractérisé en ce qu'il contient, en outre, jusqu'à 5% en poids d'un mélange additionnel de normalisation qui présente la composition suivante:

20 à 60% en poids d'acide myristique,

10 à 50% en poids d'acide cinnamique,

3 à 15% en poids de quercetine,

3 à 15% en poids de géraniol,

0 à 5% en poids d'acide férulique,

0 à 7,5% en poids de chrysine,

9 à 10% en poids d'acide caféique.


**Claims**

1. Process for producing products containing propolis by extraction of natural propolis, characterised in that choline and/or choline derivatives or aqueous solutions thereof are used for the extraction of propolis.

2. Process according to claim 1, characterised in that choline and a choline derivative are first mixed together so that the pH of the mixture obtained has a value of from 10 to 13, preferably from 11 to 12, this mixture is used for extraction and the pH of the extract is then adjusted to a value of from 6.5 to 9.5, preferably from 7 to 8.5, by means of physiologically acceptable inorganic and/or organic acids.

3. Process according to one of the claims 1 or 2, characterised in that the choline derivatives used are choline chloride and/or betaine.

4. Process according to one of the claims 1 to 3, characterised in that extraction of the propolis is carried out at a temperature range of from 50 to 70°C, preferably from 55 to 65°C and filtration of the extract is carried out at a temperature range of from 30 to 50°C, preferably from 35 to 45°C.

5. Process according to one of the claims 1, 2, 3 or 4, characterised in that the pH of the extract obtained is adjusted to a range of from 6.5 to 9.5 by means of a physiologically acceptable inorganic or organic acid.

6. Process according to claim 2 or claim 5, characterised in that phosphoric acid, sulphuric acid, ascorbic acid, citric acid, acetic acid, propionic acid, cinnamic acid, caffeic acid, ferulic acid, myristic acid or mixtures thereof are used for adjusting the pH.

7. Process according to one of the claims 1, 2, 3, 4, 5 or 6, characterised in that an emulsifier is used for extraction.

8. Product containing propolis, characterised in that it contains from 5 to 40% by weight of propolis and from 60 to 95% by weight of choline and/or choline derivatives, based on the mixture of propolis, choline and/or choline derivatives, and optionally up to 30% by weight of water, based on the whole mixture.

9. Product containing propolis, characterised in that it contains from 30 to 70% by weight of propolis extract according to claim 8, from 0 to 20% by weight of active charcoal, from 20 to 50% by weight of cocoa, coffee or malt powder and/or extract or vegetable and/or fruit concentrate, from 0 to 25% by weight of sugar and from 0 to 5% by weight of seasoning.

10. Product containing propolis according to claims 8 and 9, characterised in that it contains up to 15% by weight, based on the total mixture, of vitamins, trace elements, mineral substances, stabilisers, flavourings, amino acids, and/or pharmacologically active substances or mixtures thereof.

11. Product containing propolis according to claim 8, 9 or 10, characterised in that it contains, in addition, up to 5% by weight of a standardisation additive mixture having the following composition:

**0 061 508**

20 to 60% by weight myristic acid
10 to 50% by weight cinnamic acid
3 to 15% by weight quercetin
3 to 15% by weight geranium oil
0 to 5% by weight ferulic acid
0 to 7.5% by weight chrysin
9 to 10% by weight caffeic acid.

11